# EUROPÄISCHE PATENTSCHRIFT

(11) **EP 1 799 873 B1**
(45) Veröffentlichungstag und Bekanntmachung des Hinweises auf die Patenterteilung: **16.12.2009**
(21) Anmeldenummer: 05794900.0
(22) Anmeldetag: 13.10.2005
(51) Int. Cl.: C22C 5/02, A61K 6/04

(54) **PALLADIUM- UND KUPFER-FREIE HOCHGOLDHALTIGE DENTALLEGIERUNG**
DENTAL ALLOY WITH A HIGH GOLD CONTENT THAT IS DEVOID OF PALLADIUM AND COPPER
ALLIAGE DENTAIRE A TENEUR ELEVEE EN OR ET EXEMPT DE PALLADIUM ET DE CUIVRE

(30) Priorität: 16.10.2004 DE 102004050594
(43) Veröffentlichungstag der Anmeldung: 27.06.2007
(73) Patentinhaber: DeguDent GmbH, 63457 Hanau-Wolfgang (DE)
(72) Erfinder: STEINKE, Rudi, 63457 Hanau (DE); BAUER, Jochen, 63741 Aschaffenburg (DE); VÖLKL, Lothar, 63773 Goldbach (DE); HATHAWAY, Doris, 63457 Hanau (DE); KLAUS, Angela, 63456 Hanau (DE); SCHUSTER, Martin, 63825 Schöllkrippen (DE)
(74) Vertreter: Stoffregen, Hans-Herbert
(86) Internationale Anmeldenummer: PCT/EP2005/010992
(87) Internationale Veröffentlichungsnummer: WO 2006/040145

(56) Entgegenhaltungen:
- DE-A1- 2 424 575
- DE-A1- 2 755 913
- DE-A1- 10 033 445
- DE-C1- 10 042 316
- US-A- 4 062 676
- FISCHER J: "Effect of small additions of Ir on properties of a binary Au-Ti alloy" DENTAL MATERIALS, Bd. 18, 2002, Seiten 331-335, XP002362873

## Beschreibung

Die Erfindung bezieht sich auf eine Palladium- und Kupfer-freie hochgoldhaltige Dentallegierung, insbesondere aufbrennfähige Dentallegierung, zur Herstellung von Zahnersatz wie Kronen, Brücken, Inlays oder Onlays, enthaltend zumindest Platin sowie zumindest einen Kornfeiner.

Palladium-Kupfer-freie Legierungen, so genannte Bio-Legierungen, werden insbesondere bei empfindlichen Patienten eingesetzt. Nachteil entsprechender Legierungen ist die geringere mechanische Stabilität insbesondere beim keramischen Brand im Vergleich zu solchen hochgoldhaltigen Dentallegierungen, die Palladium und Kupfer enthalten. So ist festzustellen, dass entsprechende Palladium-Kupfer-freie Legierungen zu Verzügen neigen. Diese werden durch so genanntes Kriechen der Legierung bei hohen Temperaturen bedingt, wobei das Kriechen an den Korngrenzen lokalisiert ist.

Um eine Steigerung der Hochtemperaturstabilität bei Pd-Cu-freien hochgoldhaltigen Dentallegierungen zu erreichen, sind Ausscheidungen an den Korngrenzen wünschenswert. Hauptbestandteile der Ausscheidungsphasen enthalten hochschmelzende Elemente, wobei signifikant messbare Anteile an Gold in den Ausscheidungen nicht enthalten sind. Platin neigt zu undefinierten Ausscheidungen, die sich durch inhomogene Verteilung der Phase und undefinierte Morphologie äußern. Im Extremfall führt dies zu einer so genannten "Sternchen"-Bildung. Unabhängig hiervon treten aufgrund der undefinierten Ausscheidung Verzüge auf. Auch wird die Polierbarkeit und Fräsbarkeit der Legierung beeinträchtigt. Optisch verliert die Legierung an goldener Farbe.

Aus der EP-A-1 193 320 ist eine aufbrennfähige, hocligoldhaltige Dentallegierung bekannt, die 80,0 bis 86,5 % Gold, 7,1 bis 13 % Platin, 0,1 bis 8 % Palladium, 0 bis 1,2 % Silber, 0,7 bis 3,5 % Zink, 0,0 bis 1,0 % Eisen, Iridium, Ruthenium, Rhodium, Tantal, Mangan, Rhenium, Niob, 0 bis 3,5 % Zinn, Indium, Gallium oder 0 bis 0,5 % Kupfer enthält. Nachteil der entsprechenden Legierung ist der Palladium-Anteil, so dass ein Einsatz als Bio-Legierung nicht möglich ist.

Eine Palladium-, Gallium- und Kupfer-freie Dentallegierung mit hohem thermischen Ausdehnungskoeffizienten wird in der US-A-5,423,680 beschrieben. Die Legierung kann 50 bis 75 % Au, 8 bis 9 % Pt, 12,4 bis 38 % Ag, 2 % In, 1bis 2 % Mn, 4 % Sn, 1 bis 1,9 % Zn, 0,05 % Ir und 0,05 % CaB₆ enthalten. Als weitere Bestandteile sind Platin, Niob und Tantal genannt.

Eine Dentaledelmetalllegierung nach der DE-A-31 32 143 enthält 70 - 80 % Gold, 1 10 % Platin, 5 - 15 % Palladium, 0,1 - 5 % Zinn, 0- 5 % Indium, 0 - 2 % Zink, 0,1 9 % Silber oder Kupfer, 0,0 - 2 % Iridium, Rhenium oder Ruthenium sowie 0,1 - 3 % Kobalt oder Chrom oder Gallium oder Molybdän oder Niob oder Tantal oder Vanadium.

Der DE-A-100 33 445 ist eine hochgoldhaltige Dentallegierung zu entnehmen, die kupferfrei sein kann. Dabei setzt sich die Legierung nach dem Beispiel 1 aus 77,6 Gew.-% Gold, 19,6 Gew.-% Platin, 2,1 Gew.-% Zink, 0,6 Gew.-% Tantal und 0,1 Gew.-% Iridium zusammen.

Eine hochgoldhaltige Legierung nach der DE-A-27 46 525 enthält maximal 0,8 Gew.-% Tantal und zumindest 0,5 Gew.-% Rhodium.

Der DE-A-24 24 575 ist eine Aufbrenngoldlegierung zu entnehmen, die neben 80 bis 90 Gew.-% Gold, 5 bis 15 Gew.-% Platin, 0,1 bis 2 Gew.-% In, 0 bis 2 Gew.-% Zinn und 0,05 bis 0,5 Gew.-% Ir noch 0,5 bis 3 Gew.-% Rhodium enthält. Dabei können anstelle von Rhodium oder neben Rhodium jeweils 0,1 bis 2 Gew.-% Tantal und/oder Wolfram zugegen sein.

Eine Aufbrenngoldlegierung nach der US-A-4 062 676 enthält 60 % - 90 % Au, 5 % - 35 % Pt, 0,1 % - 3% In, 0% - 10% Pd, 0% - 3% Sn, 0,5% - 3% Rh, 0,1% - 2% Ta und/oder W und 0,3 % - 2 % Zn, wobei das Gewichtsverhältnis der Metalle der Platingruppe zu Zink zu Ta und/oder W sich verhält wie 15 - 30 : 1 : 0,5 - 1,3.

Der vorliegenden Erfindung liegt die Aufgabe zugrunde, eine Palladium- und Kupfer-freie hochgoldhaltige Dentallegierung der eingangs genannten Art so weiterzubilden, dass eine hohe mechanische Stabilität, insbesondere eine reproduzierbare Hochtemperaturstabilität gegeben ist.

Erfindungsgemäß wird das Problem im Wesentlichen dadurch gelöst, dass die Dentallegierung aus 75 bis 95 Gew.-% Au, 5 bis 20 Gew.-% Pt, 0,5 bis 3,5 Gew.-% Zn und/oder Sn und/oder In, 0,1 bis 0,8 Gew.-% eines Elements einer Gruppe 1 sowie einem einzigen Komfeiner einer Gruppe II besteht, dass der Gewichtsanteil des Elements der Gruppe I 2- bis 6-fach größer als der des einzigen Kornfeiners der Gruppe II ist und dass Nb oder Ta oder Ti oder V ein Element der Gruppe I und Ir oder Rh ein Kornfeiner der Gruppe II ist, wobei bei Vorliegen von Nb der Komfeiner Ir, bei Vorliegen von Ta der Komfeiner Rh und bei Vorliegen von Ti oder V der Komfeiner Ir oder Rh ist.

Insbesondere setzt sich die Dentallegierung aus 80 bis 91 Gew.% Au, 7,5 bis 18 Gew.-% Pt, 1 bis 2,5 Gew.-% Zn und/oder Sn und/oder In sowie 0,2 bis 0,6 Gew.-% eines Elements der Gruppe I zusammen, wobei der Gewichtsanteil des Elements der Gruppe I ebenfalls 2- bis 6-fach größer als der des einzigen Kornfeiners der Gruppe II ist.

Besonders bevorzugt besteht eine erfindungsgemäße Dentallegierung aus der Zusammensetzung 80 bis 84 Gew.-% Au, 14 bis 17 Gew.% Pt, 1,5 bis 2,2 Gew.-% In und/oder Sn und/oder Zn, 0,3 bis 0,5 Gew.-% eines der Elemente der Gruppe I sowie einem einzigen Komfeiner der Gruppe II, wobei der Gewichtsanteil des Elements der Gruppe I 2- bis 6-fach größer als der einzige Kornfeiner der Gruppe II ist.

Die Elemente der Legierung ergeben eine Gesamtsumme von 100 %.

Bevorzugterweise ist Niob das Element der Gruppe I und Iridium der einzige Komfeiner, wobei insbesondere der Gewichtsanteil von Iridium 0,05 bis 0,15 Gew.% beträgt. Insbesondere zeichnet sich die Palladium- und Kupfer-freie hochgoldhaltige Dentallegierung dadurch aus, dass die Dentallegierung aus oder in etwa aus 81,6 Gew.-% Au, aus oder in etwa aus 16 Gew.% Pt, aus oder in etwa aus 1,4 Gew.-% Zn, aus oder in etwa aus 0,5 Gew.-% In, aus oder in etwa aus 0,4 Gew.-% Nb und aus oder in etwa aus 0,1 Gew.-% Ir besteht.

Die inhomogene Verteilung der Phase sowie eine undefinierte Morphologie der Ausscheidung sind Folgen einer ungesteuerten, nicht kontrollierten Ausscheidungskinetik. Elemente, die im Periodensystem direkt nebeneinander, untereinander oder in Schrägbeziehung zueinander stehen, besitzen eine physikalische und chemische Ähnlichkeit. Eine diesbezügliche chemische/physikalische Affinität führt nach der Keimbildung zu einer schnellen Anlagerung von Atomen und mit der Folge einer Vorzugswachstumsrichtung im Gefüge, die im Extremfall durch die "Sternchenbildung" oder durch dendritische Ausscheidungen in Erscheinung tritt.

Eine diesbezügliche Kinetik der Ausscheidungsbildung kann überraschend dadurch gehemmt werden, dass hochschmelzende Elemente verwendet werden, die zwar an der Ausscheidungsphase beteiligt sind, jedoch chemisch bzw. physikalisch unähnlich sind. Überraschenderweise hat sich insbesondere gezeigt, dass die erfindungsgemäße hochgoldhaltige Dentallegierung bei der Anwesenheit von Niob und des einzigen Kornfeiners in Form von Iridium unter Berücksichtigung des Verhältnisses der Elemente Niob und Kornfeiner eine homogene definierte Ausscheidung an den Korngrenzen zeigt, so dass die gewünschte mechanische Stabilität sowie Hochtemperaturstabilität erzielbar sind.

Bei hochgoldhaltigen Palladium-Kupfer-freien Dentallegierungen erfüllen folglich Iridium (Ordnungszahl 77; Konfiguration 4f14, 5d7, 6s2) und Niob (Ordnungszahl 41; Konfiguration 4d4, 5s1) die Voraussetzungen, um eine ungesteuerte, nicht kontrollierte Ausscheidungskinetik zu unterbinden.

Eine homogene definierte Ausscheidung an den Korngrenzen bei Palladium- und Kupfer-freien hochgoldhaltigen Dentallegierungen zeigt sich jedoch auch dann, wenn anstelle von Niob Tantal, Vanadium oder Titan mit Gewichtsanteilen zum Einsatz gelangt, die dem von Niob entspricht. Im Falle von Tantal wird als einziger Kornfeiner Rhodium benutzt. Wird Niob durch Vanadium oder Titan ersetzt, so kann als einziger Kornfeiner Iridium oder Rhodium eingesetzt werden. Unabhängig hiervon ist das Mengenverhältnis von Tantal zu Rhodium bzw. Vanadium zu Iridium oder Rhodium bzw. Titan zu Iridium bzw. Rhodium entsprechend dem zwischen Niob und Iridium.

Schliffbilder erfindungsgemäßer Legierungen zeigen eine sehr feine Ausscheidung an den Korngrenzen über einen weit variierenden Bereich an Gold- und Platinanteilen, wobei insbesondere der Gewichtsanteil von Gold zwischen 80 und 88 % und der von Platin zwischen 10 und 18 % liegt:

Aufgrund der kinetischen Kontrolle der Ausscheidung zeigt die Legierung neben einer hohen Verzugsstabilität eine gute Polierbarkeit, Fräsbarkeit und Farbstabilität.

Palladium- und Kupfer-freie hochgoldhaltige Legierungen der erfindungsgemäßen Zusammensetzung sind in der nachstehenden Tabelle I unter Nr. 5.6 und 8 aufgeführt.

| **Nr.** | **Au** | **Pt** | **Zn** | **In** | **Ta** | **Nb** | **Ir** | **Rh** |
|---|---|---|---|---|---|---|---|---|
| 1 | 81,3 | 16 | 1,7 | 0,5 | - | 0,2 | - | 0,3 |
| 2 | 85,2 | 11,9 | 1,3 | 0,7 | - | 0,4 | 0.1 | 0,4 |
| 3 | 85,3 | 12,2 | 1,4 | 0,7 | 0,2 | - | 0,2 | - |
| 4 | 81,4 | 16 | 1,7 | 0,5 | - | - | 0,1 | 0,3 |
| 5 | 86 | 11,6 | 1,4 | 0,5 | - | 0,4 | 0,1 | - |
| 6 | 81,6 | 16 | 1,4 | 0,5 | - | 0,4 | 0,1 | - |
| 7 | 84,5 | 12,6 | 1 | 1 | - | 0,3 | 0,1 | 0,5 |
| 8 | 81,5 | 16,0 | 1,4 | 0,5 | 0,4 | - | - | 0,2 |

Die unter den Nr. 1 - 4 und 7 aufgeführten hochgoldhaltigen Legierungen sind ebenfalls Palladium- und Kupfer-frei, also Biolegierungen. In diesen sind jedoch entweder mehr als ein Komfeiner (Legierung Nr. 2, Nr. 7) oder Elemente der Gruppe I und ein Komfeiner enthalten, die ein von der erfindungsgemäßen Lehre abweichendes Gewichtsverhältnis aufweisen (Legierung Nr. 1, Nr. 3). Bei der Legierung Nr. 4 findet sich kein Element der Gruppe 1.

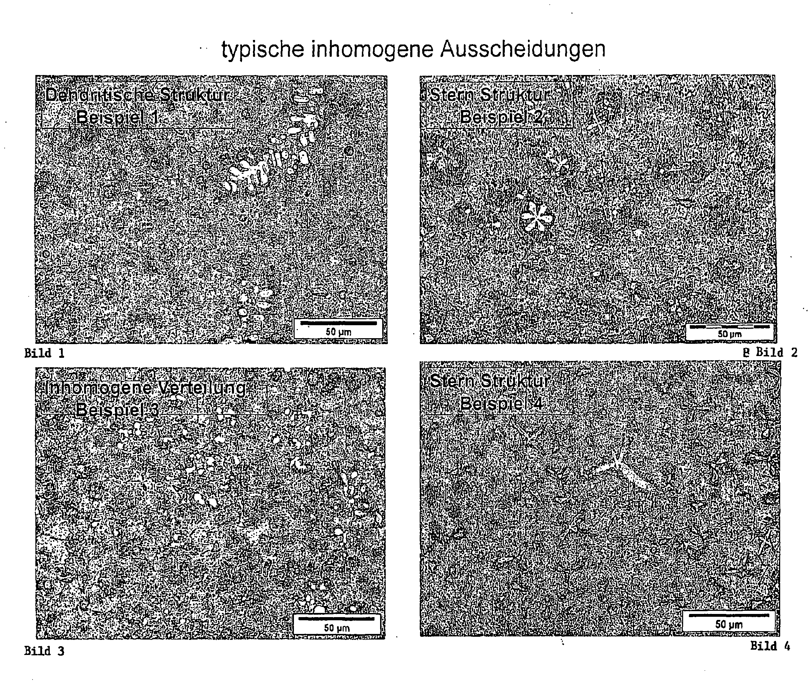

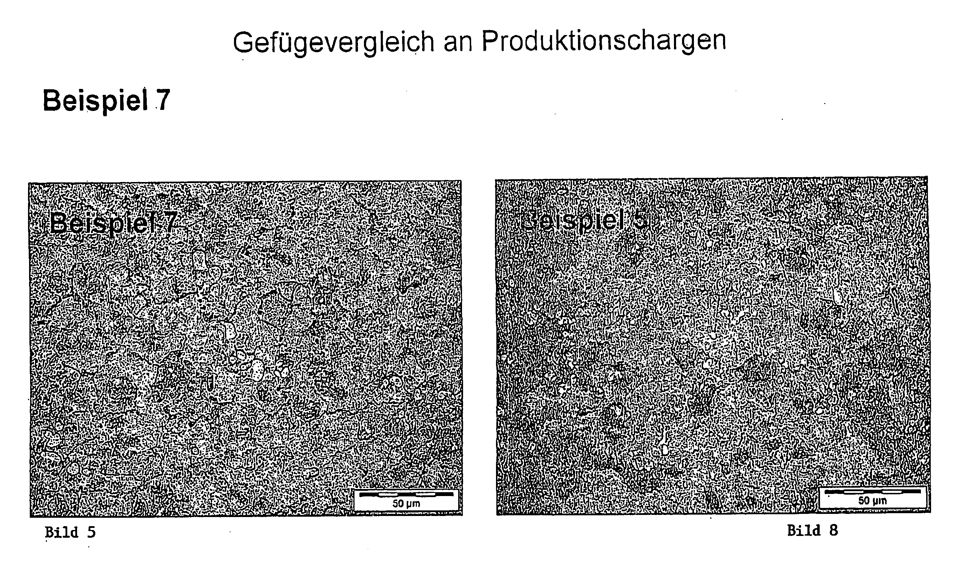

Wie Schliffbilder der Legierung Nr. 1 (Bild 1), der Legierung Nr. 2 (Bild 2), der Legierung Nr. 3 (Bild 3), der Legierung Nr. 4 (Bild 4) und der Legierung Nr. 7 (Bild 5) verdeutlichen, treten bei Palladium- und Kupfer-freien hochgoldhaltigen Legierungen, die nicht eine erfindungsgemäße Zusammensetzung aufweisen, undefinierte Ausscheidungen an den Korngrenzen auf, die zu den dendritischen Strukturen (Bild 1), Stemstrukturen (Bild 2, Bild 4) bzw. inhomogenen Verteilungen (Bild 3, Bild 5) führen.

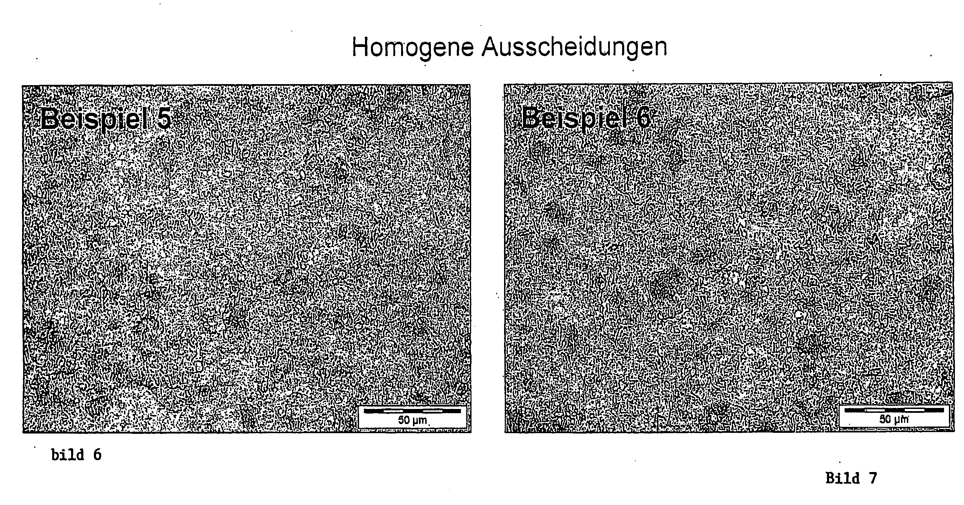

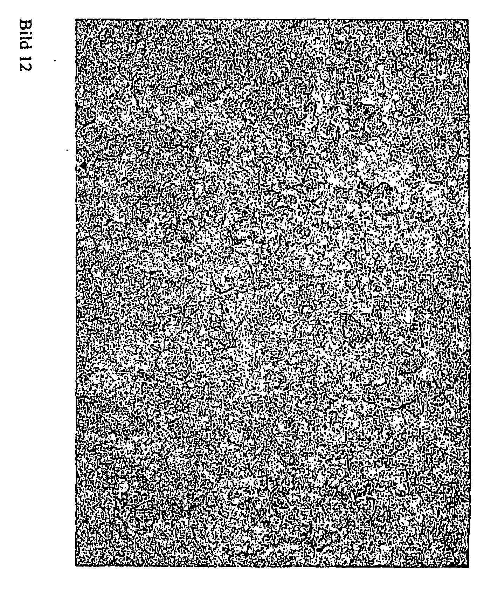

Demgegenüber zeigen Schliffbilder der Legierung Nr. 5 (Bild 6), der Legierung Nr. 6 (Bild 7) und der Legierung 8 (Bild 12), die jeweils eine erfindungsgemäße Zusammensetzung aufweisen, homogene Ausscheidungen, die zu einer Hochtemperaturstabilität, die insbesondere beim Aufbrennen von Keramik wichtig ist, eine hohe Verzugsstabilität, eine gute Polierbarkeit und Fräsbarkeit führen. Auch zeigen entsprechende erfindungsgemäße Legierungen eine gute Farbstabilität.

In Bild 8 ist des Weiteren ein Schliffbild der Legierung Nr. 5 zu entnehmen, die aus einer Produktionscharge stammt. Entsprechend gilt für das Schliffbild die Legierung Nr. 7 (Bild 5). Man erkennt bei dem Schliffbild der Legierung Nr. 5 (Bild 8) homogene Ausscheidungen. Demgegenüber zeigt das Schliffbild der Legierung Nr. 7 (Bild 5), die neben Niob zwei Komfeiner enthält, inhomogene Ausscheidungen.

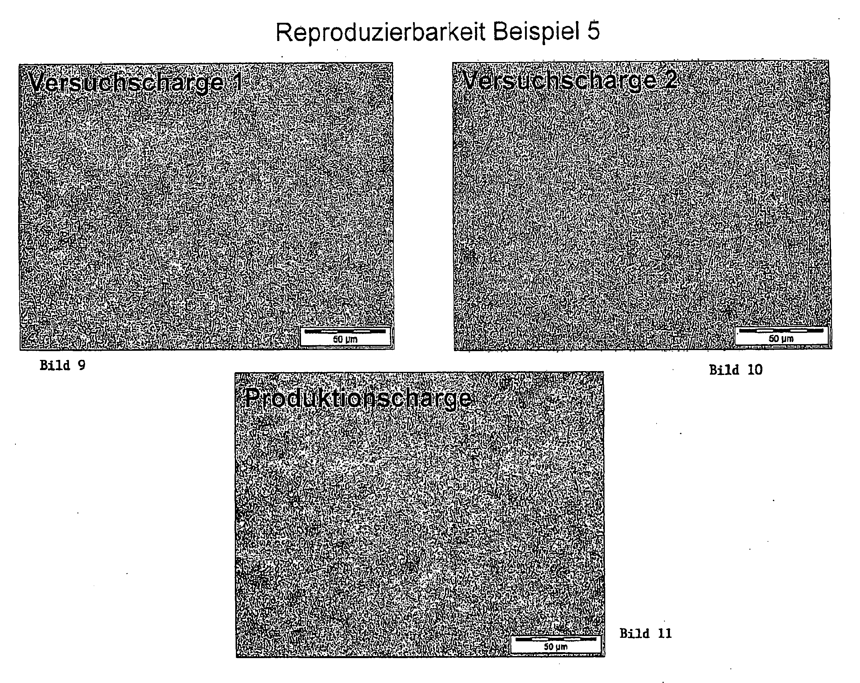

Anhand der Schlifibilder gemäß Bild 9, Bild 10 und Bild 11 ist die Reproduzierbarkeit der homogenen Ausscheidung durch das im Beispiel der Legierung Nr. 5 erfindungsgemäß vorgegebene Verhältnis von Niob und Iridium (4:1) erkennbar. So sind sowohl bei Legierungen der Zusammensetzung Nr. 5 aus Versuchschargen (Bild 9, Bild 10) als auch bei einer Legierung gemäß der Zusammensetzung Nr. 5 aus einer Produktionscharge fein verteilte Ausscheidungen an den Korngrenzen feststellbar, die zu den hervorzuhebenden Materialeigenschaflen der erfindungsgemäßen Palladium- und Kupfer-freien hochgoldhaltigen Legierung führen.

## Patentansprüche

1. Palladium- und Kupfer-freie hochgoldhaltige Dentallegierung, insbesondere aufbrennfähige Dentallegierung, zur Herstellung von Zahnersatz wie Kronen, Brücken, Inlays oder Onlays, enthaltend zumindest Platin sowie zumindest einen Kornfeiner,
**dadurch gekennzeichnet,**
**dass** die Dentallegierung aus 75 bis 95 Gew.% Au, 5 bis 20 Gew.-% Pt, 0,5 bis 3,5 Gew.-% Zn und/oder Sn und/oder In, 0,1 bis 0,8 Gew.-% eines Elements einer Gruppe I sowie einem einzigen Komfeiner einer Gruppe II besteht,
**dass** der Gewichtsanteil des Elements der Gruppe I 2- bis 6-fach größer als der des einzigen Kornfeiners der Gruppe II ist und
**dass** Nb oder Ta oder Ti oder V ein Element der Gruppe I und Ir oder Rh ein Komfeiner der Gruppe II ist,
wobei bei Vorliegen von Nb der Kornfeiner Ir, bei Vorliegen von Ta der Kornfeiner Rh und bei Vorliegen von Ti oder V der Kornfeiner Ir oder Rh ist.

2. Dentallegierung nach Anspruch 1,
**dadurch gekennzeichnet,**
**dass** die Dentallegierung aus 80 bis 91 Gew.-% Au, 7,5 bis 18 Gew.-% Pt, 1 bis 2,5 Gew.-% Zn und/oder Sn und/oder In sowie 0,2 bis 0,6 Gew.-% eines Elements der Gruppe I sowie dem einzigen Komfeiner der Gruppe II besteht, wobei der Gewichtsanteil des Elements der Gruppe I 2- bis 6-fach größer als der des einzigen Kornfeiners der Gruppe II ist.

3. Dentallegierung nach Anspruch 1 oder 2,
**dadurch gekennzeichnet,**
**dass** die Dentallegierung aus 80 bis 84 Gew.-% Au, 14 bis 17 Gew.-% Pt, 1,5 bis 2,2 Gew.-% In und/oder Sn und/oder Zn, 0,3 bis 0,5 Gew.-% eines der Elemente der Gruppe I sowie einem einzigen Kornfeiner der Gruppe 11 ist, wobei der Gewichtsanteil des Elements der Gruppe I 2- bis 6-fach größer als der einzige Komfeiner der Gruppe II besteht.

4. Dentallegierung nach zumindest einem der vorhergehenden Ansprüche,
**dadurch gekennzeichnet,**
**dass** der Gewichtsanteil von Iridium 0,05 bis 0,15 Gew.-% beträgt.

5. Dentallegierung nach zumindest einem der vorhergehenden Ansprüche,
**dadurch gekennzeichnet,**
**dass** die Dentallegierung aus 81,6 Gew.% Au, 16 Gew.% Pt, 1,4 Gew.% Zn, 0,5 Gew.-% In, 0,4 Gew.-% Nb und 0,1 Gew.-% Ir besteht.

## Claims

1. Palladium- and copper-free high-gold-content dental alloy, in particular a fire-on dental alloy, for the production of dental prostheses such as crowns, bridges, inlays, or onlays, containing at least platinum and at least one particle refiner,
**characterized in**
**that** the dental alloy consists of 75 to 95 wt. % Au, 5 to 20 wt. % Pt, 0.5 to 3.5 wt. % Zn and/or Sn and/or In, 0.1 to 0.8 wt. % of an element of a group I, as well as a single particle refiner of a group II,
**that** the weight proportion of the group I element is 2 to 6 times greater than that of the single particle refiner of group II, and
**that** Nb or Ta or Ti or V is an element of group I and Ir or Rh is a particle refiner of group II,
in the presence of Nb the particle refiner being Ir, in the presence of Ta the particle refiner being Rh, and in the presence of Ti or V the particle refiner being Ir or Rh.

2. Dental alloy according to claim 1,
**characterized in**
**that** the dental alloy consists of 80 to 91 wt. % Au, 7.5 to 18 wt. % Pt, 1 to 2.5 wt. % Zn, and/or Sn and/or In as well as 0.2 to 0.6 wt. % of a group I element as well as the single particle refiner of group II, whereby the weight proportion of the group I element is 2 to 6 times greater than that of the single particle refiner of group II.

3. Dental alloy according to claim 1 or 2,
**characterized in**
**that** the dental alloy consists of 80 to 84 wt. % Au, 14 to 17 wt. % Pt, 1.5 to 2.2 wt. % In, and/or Sn and/or Zn, 0.3 to 0.5 wt. % of a group I element, as well as a single particle refiner of group II, the weight proportion of the group I element being 2 to 6 times greater than that of the single particle refiner of group II.

4. Dental alloy according to at least one of the preceding claims,
**characterized in**
**that** the weight proportion of iridium is 0.05 to 0.15 wt. %.

5. Dental alloy according to at least one of the preceding claims,
**characterized in**
**that** the dental alloy consists of 81.6 wt. % Au, 16 wt. % Pt, 1.4 wt. % Zn, 0.5 wt. % In, 0.4 wt. % Nb, and 0.1 wt. % Ir.

## Revendications

1. Alliage dentaire à forte teneur en or exempt de palladium et de cuivre, en particulier alliage dentaire céramo-métallique, destiné à la confection de prothèses dentaires telles que couronnes, bridges, inlays ou onlays, contenant au moins du platine ainsi qu'au moins un affineur de grain,
**caractérisé en ce**
**que** l'alliage dentaire est constitué de 75 à 95 % en poids d'Au, de 5 à 20 % en poids de Pt, de 0,5 à 3,5 % en poids de Zn et/ou de Sn et/ou d'In, de 0,1 à 0,8 % en poids d'un élément d'un groupe I ainsi que d'un unique affineur de grain d'un groupe II,
**que** la part en poids de l'élément du groupe I est 2 à 6 fois supérieure à celle de l'unique affineur de grain du groupe II et,
**que** le Nb ou le Ta ou le Ti ou le V est un élément du groupe I et que l'Ir ou le Rh est un affineur de grain du groupe II,
sachant que l'affineur de grain est de l'Ir en présence de Nb, du Rh en présence de Ta et de l'Ir ou du Rh en présence de Ti ou de V.

2. Alliage dentaire selon la revendication 1,
**caractérisé en ce**
**que** l'alliage dentaire est constitué de 80 à 91% en poids d'Au, de 7,5 à 18 % en poids de Pt, de 1 à 2,5 % en poids de Zn et/ou de Sn et/ou d'In ainsi que de 0,2 à 0,6 % en poids d'un élément du groupe I et de l'unique affineur de grain du groupe II, sachant que la part en poids de l'élément du groupe I est 2 à 6 fois supérieure à celle de l'unique affineur de grain du groupe II.

3. Alliage dentaire selon la revendication 1 ou 2,
**caractérisé en ce**
**que** l'alliage dentaire est constitué de 80 à 84 % en poids d'Au, de 14 à 17 % en poids de Pt, de 1,5 à 2,2 % en poids d'In et/ou de Sn et/ou de Zn, de 0,3 à 0,5 % en poids d'un des éléments du groupe I ainsi que d'un unique affineur de grain du groupe II, sachant que la part en poids de l'élément du groupe I est 2 à 6 fois supérieure à celle de l'unique affineur de grain du groupe II.

4. Alliage dentaire selon au moins l'une des revendications précédentes,
**caractérisé en ce**
**que** la part en poids d'iridium est comprise entre 0,05 et 0,15 %.

5. Alliage dentaire selon au moins l'une des revendications précédentes,
**caractérisé en ce**
**que** l'alliage dentaire est constitué de 81,6 % en poids d'Au, de 16 % en poids de Pt, de 1,4 % en poids de Zn, de 0,5 % en poids d'In, de 0,4 % en poids de Nb et de 0,1 % en poids d'Ir.
